# EUROPÄISCHE PATENTSCHRIFT

(11) **EP 2 751 255 B1**
(45) Veröffentlichungstag und Bekanntmachung des Hinweises auf die Patenterteilung: **21.10.2020**
(21) Anmeldenummer: 12756428.4
(22) Anmeldetag: 31.08.2012
(51) Int. Cl.: C12N 1/12, C12M 1/00

(54) **VERFAHREN UND VORRICHTUNG ZUR GEZIELTEN EINSPEISUNG VON GASEN ODER GASGEMISCHEN IN EINE FLÜSSIGKEIT, SUSPENSION ODER EMULSION IN EINEM PHOTOBIOREAKTOR**
METHOD AND DEVICE FOR FEEDING GASES OR GAS MIXTURES INTO A LIQUID, SUSPENSION, OR EMULSION IN A PHOTOBIOREACTOR IN A SPECIFIC MANNER
PROCÉDÉ ET DISPOSITIF POUR L'INTRODUCTION CIBLÉE DE GAZ OU DE MÉLANGES GAZEUX DANS UN LIQUIDE, UNE SUSPENSION OU UNE ÉMULSION PRÉSENTS DANS UN PHOTOBIORÉACTEUR

(30) Priorität: 01.09.2011 DE 102011081979
(43) Veröffentlichungstag der Anmeldung: 09.07.2014
(73) Patentinhaber: GICON Grossmann Ingenieur Consult GmbH, 01219 Dresden (DE)
(72) Erfinder: COTTA, Fritz, 06667 Wolfen (DE); GROSSMANN, Jochen, 01187 Dresden (DE); MATSCHKE, Martin, 07973 Greiz (DE); KOETZ, Andreas, 06849 Dessau-Roßlau (DE)
(74) Vertreter: Kailuweit & Uhlemann Patentanwälte Partnerschaft mbB
(86) Internationale Anmeldenummer: PCT/EP2012/066965
(87) Internationale Veröffentlichungsnummer: WO 2013/030340

(56) Entgegenhaltungen:
- WO-A1-02/099032
- WO-A1-2009/149519
- WO-A1-2011/022349
- WO-A2-2010/125199
- WO-A2-2011/048108
- JP-A- 1 300 021
- US-A1- 2008 160 591
- US-A1- 2008 286 851

## Beschreibung

Die Erfindung betrifft ein Verfahren und eine Vorrichtung zur gezielten Einspeisung von Gasen oder Gasgemischen in eine Flüssigkeit, Suspension oder Emulsion in einem Photobioreaktor Kohlenstoffdioxid fällt bei mehreren technischen Verfahren, insbesondere bei der Verbrennung von fossilen Brennstoffen (Kohle, Koks) mit überschüssiger Luft, beim Kalkbrennprozess oder bei der Synthesegasherstellung (Kohlevergasung, Steam-Reforming) als Abprodukt an. Zum Einsatz von Synthesegas, z.B. in der Ammoniak- oder Methanolsynthese wird dieses, beispielsweise nach dem Rectisolverfahren gewaschen, wobei Kohlenstoffdioxid in sehr reiner Form gewonnen werden kann. Zum leichteren Transport wird Kohlenstoffdioxid am Ort der Herstellung verdichtet, verflüssigt oder gefroren und als Trockeneis niedergeschlagen.

Wenngleich es einige Verwendungsmöglichkeiten für Kohlenstoffdioxid z.B. in der Lebensmittelindustrie (Trockeneis-Kühlung, kohlensäurehaltige Getränke, Kohlensäure-Maischung), in der chemischen Industrie (Harnstoffsynthese, Kolbe-Schmitt-Reaktion zu Salicylsäure), beim Schweißen als Schutzgas oder für den Einsatz in Nebelmaschinen gibt, liegt der Verbrauch deutlich unter der erzeugten Menge. Aufgrund der zunehmenden Klimaveränderung durch das Treibhausgas Kohlenstoffdioxid und der wachsenden Weltbevölkerung gewannen in den letzten Jahren vor allem Verfahren zur stoffwirtschaftlichen Nutzung von Kohlenstoffdioxid erheblich an Bedeutung. Aufgrund der im Vergleich zu Landpflanzen etwa 100-mal höheren Bildungsrate von Biomasse ist die Mikroalgenherstellung derzeit das erfolgversprechendste Verfahren für eine stoffwirtschaftliche und/oder energetische Nutzung. Aus Kohlenstoffdioxid, Wasser und (Sonnen)-Licht wird durch Photosynthese Biomasse gebildet, die einer vielfältigen Nutzung zugeführt werden kann (z.B. Carotinoide, Lipide und/oder Proteine).

Bisher wurden weltweit zahlreiche Photobioreaktoren und Verfahrenskonzepte entwickelt, wobei sich zunehmend das geschlossene Photobioreaktionssystem durchgesetzt hat, da in diesen der Eintrag von Keimen, Pilzen, Bakterien u. a. Störstoffen nahezu ausgeschlossen wird. Im Bereich der geschlossenen Systeme zeigen durchströmte Schlauchsysteme, insbesondere Doppelschlauchsysteme deutliche Vorteile hinsichtlich Strömungstotzonen, Biobelagbildung, Fäulnis, Reinigungszeiten, Energieeffizienz, Wärmeübertragung, Wärmesteuerung und Biomassewachstum gegenüber z.B. Platten- oder Beutelsystemen. Allen geschlossenen Systemen gemein ist bisher die Zugabe von Kohlenstoffdioxid oder kohlenstoffdioxidhaltigen Gasen (Rauchgase) aus Druckbehältnissen oder druckerzeugenden Aggregaten, wie beispielsweise Kompressoren und Gebläsen. Der Einsatz von Kohlenstoffdioxid erfolgt ausschließlich unter stoffwirtschaftlichen Aspekten. Die Kühlung des Reaktionssystems erfolgt bei kleinen Anlagen mittels separaten Kühlkreislaufs (das Kühlwasser wird z. B. durch einen Thermostaten temperiert), bei großtechnischen Anlagen z. B. durch Verdunstungskühlung (Wasser wird auf das Photobioreaktionssystem gesprüht). Ein bisher noch nicht gelöstes Problem in Photobioreaktoren stellt die Biobelagbildung an den Wänden der Reaktoren dar, daraus resultieren längere Stillstandzeiten der Anlage durch kürzere Reinigungsintervalle, sowie geringere Biomasse-Wachstumsraten bis zum möglichen Komplettverlust der Biomasse, verursacht durch Fäulnisprozesse.

Weiterhin wird in derzeit üblichen Photobioreaktoren ein Biotrockenmasse-Gehalt von ca. 5 g/l erreicht. Bei höheren Konzentrationen erfolgt Flockung und Absetzen an den Reaktorwandungen, was dazu führt, dass kein Licht mehr ins Reaktorinnere gelangt.

Diesem Effekt wird durch die Verwendung von hohen Strömungsgeschwindigkeiten entgegengearbeitet, was den Einsatz von hoher Pumpenergie bedingt. Zusätzlich werden dem System Putzkörper zugesetzt. Diese erhöhen den technischen Aufwand zur Suspensionstrennung erheblich und minimieren zugleich das photoaktive Reaktionsvolumen.

Im Fall der Mikroalgenproduktion ist diese Verfahrensweise zur Erreichung wirtschaftlicher Produktionsweisen zwingend notwendig. Andernfalls erfolgt statt der gewünschten Photosynthese (Lichtreaktion) zunehmend Zellatmung (Dunkelreaktion), was letztlich zum Absterben der Kultur führen kann. Eine Begrenzung der maximalen BiotrockenmasseKonzentration auf ca. 5 g/l ergibt sich zudem durch den notwendigen Energieaufwand zur Durchmischung des Systems, um alle Mikroalgen mit ausreichend Licht zu versorgen. Weiterhin erfordert eine geringe Mikroalgenkonzentration in der Suspension einen erheblichen Energieaufwand bei der Abtrennung der Mikroalgen und deren Trocknung. Eine wirtschaftliche Verfahrensweise bei der Herstellung von Mikroalgen bedingt daher die Notwendigkeit höherer Konzentrationen der Biotrockenmasse vor den Aufarbeitungsprozessen.

In der stoffwandelnden Industrie werden eine Vielzahl von Reaktionen zur Erzeugung von Produkten genutzt, die eine intensive Durchmischung der Reaktionspartner, insbesondere bei dem Einsatz von Gasen in Flüssigkeiten, voraussetzen. Diese Arbeitsweise bedingt oft den Einsatz von komplizierten technischen Systemen bzw. ist mit einem hohen Energieeinsatz verbunden. Besonders anspruchsvoll sind solche Systeme in der Biotechnologie, wo es auf kontaminationsfreie bzw. kontaminationsarme Arbeitsweisen ankommt. Beispielsweise benötigen Photobioreaktoren ausgefeilte Systeme, um die Biobelagbildung an den Lichteintrittsflächen zu minimieren und somit überhaupt eine Produktion von Biomasse über Zeiträume von mehreren Wochen zu gewährleisten. Aus dem Stand der Technik sind hierzu verschiedene Verfahren bekannt:
Beispielsweise beschreibt die US 6,220,822 B1 einen Air-Lift Reaktor, der im Wesentlichen aus einem mit Flüssigkeit gefüllten Rohr besteht, in welches Luft eingeblasen werden kann. Die Luftblasen steigen durch das Rohr nach oben in ein Vorratsgefäß und treffen auf ein in diesem Gefäß schräg eingebautes Ablenkblech. Aufgrund der Aufwärtsbewegung der Gasblasen an der Oberfläche des Ablenkbleches wird in gleicher Richtung eine Strömung der im Vorratsgefäß befindlichen Flüssigkeit erzwungen. Neben Ablenkblechen werden auch z. B. konische Einbauten vorgeschlagen, die ebenfalls eine Strömung im Vorratsgefäß bewirken.

Die US 4,649,117 beschreibt einen Reaktor, der das Air-Lift-Prinzip zum Beispiel für eine effektivere Zellernte aus Fermentern/Bioreaktoren nutzt. Als wesentlicher Vorteil dieser Anwendung wird ohne den Einsatz mechanischer Rühraggregate eine optimale Zirkulation der Flüssigkeit bei minimaler mechanischer Scherkraft beschrieben. Das eingesetzte, komprimierte Gas besteht aus Luft mit einem Anteil Kohlendioxid von etwa 5 %. Erfindungsgemäß steigen die am Boden des Reaktors eingespeisten Gasblasen gerade nach oben. Der Reaktorinhalt beträgt etwa 5-7 Liter.

Weiterhin offenbart die US 2009/0303829 A1 die Verwendung einer flexiblen, doppelwandigen, aufblasbaren Folie in Form eines Rohres zur Luftzufuhr, das in der Bodenmitte des Vorratsbehälters verankert ist. Zugeführte Gase können Luft, Sauerstoff, Kohlendioxid oder andere sein.

Die WO 99/25657 beschreibt einen Bioreaktor mit guter Durchmischung zur aeroben Behandlung wässeriger Abfalle mit einem hohen Anteil organischer und fester Bestandteile unter Verwendung des Air-Lift Prinzips. Neben der "Air-Lift Pumpe" wird weiterhin ein Diffuser zur Verteilung der eingesetzten Gase, z.B. Sauerstoff, Stickstoff, Ammoniak eingesetzt. Die WO 99/25657 beschreibt ausschließlich die Behandlung wässeriger Abfälle, die eingesetzten Gase werden durch Redoxreaktionen umgesetzt.

Die US 7,629,167 B2 offenbart flexible Einweg-Bioreaktoren in Form von Containern/Beuteln/Säcken aus Plastik. Der flexible und austauschbare Bioreaktor wird dabei von einem soliden Gefäß, wie zum Beispiel einem Tank umschlossen. Zum Flüssigkeits- und/oder Gasaustausch werden verschiedene Fittings/Verschlüsse beschrieben. Weiterhin können Sensoren zur Überwachung des Bioreaktors eingesetzt werden. Zur Erzeugung von Strömungen im Bioreaktor können über der Gaseintrittsöffnung Ablenkbleche oder andere Einbauten genutzt werden. Eingesetzte (komprimierte/geförderte) Gase können sein Luft, Sauerstoff und/oder Kohlendioxid.

Die US 2005/0098497 A1 beschreibt einen Reaktor, der im Wesentlichen dadurch gekennzeichnet ist, dass dieser mit einer Flüssigkeit oder Suspension gefüllt ist. Im Fall des Vorliegens einer Suspension wird ein Gas über ein Tauchrohr zugegeben und mittels Diffusoren verteilt, sodass die Dichte des Flüssigkeit-Gas-Gemisches deutlich geringer wird und der Feststoff absinkt. Daraus ergeben sich die in der US 2005/0098497 A1 aufgeführten Anwendungsmöglichkeiten der fest-flüssig-Trennung. Der Trenneffekt wird dadurch erhöht, dass das Gas pulsiert zugegeben werden kann. Weiterhin sind verschiedene Modifikationen (Einbau von Filtern/Festbett in den Reaktor, Ablenkbleche, verschiedene Diffusorgeometrien) angegeben.

Die WO 2011/048108 A2 beschreibt einen Schlauch-Photobioreaktor, z. B. zur Produktion von Mikroalgen mit kegelstumpf-förmiger Kernstruktur und einem oder mehreren transparenten oder transluzenten Schläuchen. Der Schlauch ist helikal auf das Grundgerüst aufgewickelt und zeichnet sich insbesondere dadurch aus, dass dieser mindestens zwei Kammern aufweist. Mindestens eine Kammer wird von einem Kultivierungsmedium, mindestens eine von einem Wärmetauschmedium durchströmt. Das Schlauchmaterial besteht aus Kunststoffen oder Glas, vorzugsweise aus Silikonen. Dadurch wird die Biobelagbildung und damit auch Anlagenstillstandzeiten durch Reinigungsarbeiten minimiert. Im Schlauch erfolgt dabei die Förderung des Kultivierungsmediums mittels Airlift, das heißt mittels Luft oder mittels eines Luft/CO₂-Gemisches oder auch Stickstoff als Trägergas, erfolgen, welches simultan die Versorgung des Kultivierungsmediums mit CO₂ sicherstellt. Das Air-Lift-Prinzip beruht dabei auf der Einspeisung fein verteilten Gases (Mammutpumpe). Die Zuführung von CO₂ oder CO₂-haltigen Gasen kann aber auch, separat und gepulst, über ein Mischsystem oder im Vorlauf der Pumpe erfolgen und damit der Einstellung des pH-Wertes im Kultivierungsmedium dienen.

Das Dokument US 2008/0286851 A1 offenbart die gepulste Einspeisung von Gas in einen Photobioreaktor, wobei durch die gepulste Einspeisung Turbulenzen in der Flüssigkeit erzeugt werden, die eine Durchmischung der Suspension gewährleisten sollen.

Die Regulierung des pH-Wertes durch die verwendeten Gase erfordert jedoch eine große Oberfläche der Phasengrenzen zwischen diesen und der Flüssigkeit, was gemäß Stand der Technik ein möglichst fein verteiltes Gas voraussetzt.

Der Stand der Technik weist den Nachteil auf, dass neben der begrenzten Biomassekonzentration von ca. 5 g/l aufgrund von Biobelagbildung ein entsprechender Reinigungsaufwand und damit Reaktorstillstandzeiten oder ein erhöhter Aufwand bei der Biotrockenmassetrennung, wie etwa bei Zugabe von Putzkörpern notwendig ist.

Ein Verfahren, welches eine Erhöhung der Konzentration der Biotrockenmasse über 5 g/l und eine gleichzeitige Reduzierung der Biobelagbildung in einfacher Weise und somit eine Reduktion von Stillstandzeiten von Bioreaktoren gewährleistet, wäre in hohem Maße wünschenswert.

Die Aufgabe der vorliegenden Erfindung besteht daher darin, ein Verfahren zu beschreiben, welches die Nachteile aus dem Stand der Technik überwindet und in einfacher Weise eine Erhöhung der Biomassekonzentration über 5 g/l bei gleichzeitiger Verminderung der Biobelagbildung ermöglicht.

Die Aufgabe wird durch ein Verfahren gemäß Anspruch 1 gelöst. Vorteilhafte Ausgestaltungen sind in den abhängigen Ansprüchen angegeben. Die Aufgabe wird auch durch eine Vorrichtung gemäß Anspruch 12 gelöst. Vorteilhafte Ausgestaltungen sind in den abhängigen Ansprüchen angegeben.

Erfindungsgemäß wird die Aufgabe durch ein Verfahren zur gezielten Einspeisung von Gasen oder Gasgemischen in eine Flüssigkeit, Suspension oder Emulsion in einen Photobioreaktor gelöst. Dabei wird das Gas- oder Gasgemisch in gezielter Menge und/oder zu definierten Zeitpunkten eingespeist, wobei ein Pulsationseffekt erhalten wird. Dadurch wird mittels einer Entspannung des eingespeisten Gases oder Gasgemisches eine Triebkraft erzeugt wird, wobei durch die gewonnenen Triebkraft in der Flüssigkeit, Suspension oder Emulsion starke Turbulenzen erzeugt werden, wodurch Wandanhaftungen am Photobioreaktor unterbunden werden. Die so in der Flüssigkeit, Suspension oder Emulsion erzeugten Turbulenzen ermöglichen somit eine Durchmischung der gelösten Bestandteile als auch Feststoffe. Unter einer Entspannung des eingespeisten Gases oder Gasgemisches wird im Sinne der vorliegenden Patentanmeldung die Abkühlung des Gases oder Gasgemisches beim Übertritt von einem Bereich hohen Druckes in einen Bereich niederen Druckes verstanden, wobei die dabei entstehende Kälte erfindungsgemäß zur Kühlung verwendet wird.

Durch die erzeugten Pulsationen ist neben der so kontinuierlich erzeugten Triebkraft auch eine Durchmischung der Flüssigkeit, Suspension oder Emulsion gegeben. Dadurch werden insbesondere nachteilige Wandanhaftungen im Reaktor unterbunden. Unter Pulsation wird im Sinne der vorliegenden Patentanmeldung die Einspeisung von zeitgetakteten Gasströmen oder Gasgemischen in strömende Flüssigkeiten, Suspensionen oder Emulsionen verstanden. Gegenüber dem Stand der Technik wird das Gas dabei nicht fein verteilt, sondern in einem Stoß als Gasblase eingespeist.

In einer weiteren Ausführungsform der Erfindung wird die Flüssigkeit, Suspension oder Emulsion pulsierend gefördert. Dadurch kann der durch pulsierende Einspeisung des Gases oder Gasgemisches auftretende Pulsationseffekt verstärkt werden, wenn zur Gaseinspeisung die Förderung der Flüssigkeit, Suspension oder Emulsion ebenfalls pulsierend, z. B. mit einer Membranpumpe erfolgt. Durch Anpassung der Gaseinspeisung bzw. der pulsierenden Förderung der Flüssigkeit können so effektiv die in der Flüssigkeit, Suspension oder Emulsion entstehenden Turbulenzen entsprechend den Anforderungen eingestellt werden. Dadurch kann eine Minimierung auftretender Scherkräfte bei gleichzeitig maximaler Durchmischung der Flüssigkeit, Suspension oder Emulsion realisiert werden.

In einer weiteren Ausführungsform der Erfindung wird das Gas oder Gasgemisch mit einer hohen inneren Energie eingespeist. Unter der inneren Energie wird im Sinne der vorliegenden Patentanmeldung die Energie verstanden, die das eingesetzte Gas oder Gasgemisch aufgrund seiner Verdichtung beinhaltet und ähnlich wie bei einer Gas- oder Wärmekraftmaschine in mechanische Arbeit umgewandelt werden kann.

In einer weiteren Ausführungsform der Erfindung wird das Gas oder Gasgemisch verdichtet, zumindest teilweise kondensiert oder zumindest teilweise gefroren eingesetzt. Dadurch weist das Gas oder Gasgemisch eine im Vergleich zum Normzustand höhere innere Energie auf. Zudem kann das Gas oder Gasgemisch neben der Erzeugung eines Pulsationseffektes auch zur Kühlung der Flüssigkeit, Suspension oder Emulsion verwendet werden.

IErfindungsgemäß wird durch vollständige oder teilweise Nutzung der inneren Energie des eingespeisten Gases oder Gasgemisches die geförderte Flüssigkeit, Suspension oder Emulsion gekühlt. Dadurch kann vorteilhafter Weise neben der Erzeugung eines Pulsationseffektes auch eine Kühlung der Flüssigkeit, Suspension oder Emulsion mittels des eingespeisten Gases oder Gasgemisches erfolgen. Infolgedessen leistet die Einspeisung des Gases oder Gasgemisches einen Beitrag sowohl zum Stoff- als auch zum Energiekreislauf des Reaktors.

In einer weiteren Ausführungsform der Erfindung wird durch vollständige oder teilweise Nutzung der inneren Energie des eingespeisten Gases oder Gasgemisches die Flüssigkeit pulsierend gefördert. Dabei wird die innere Energie des eingespeisten Gases oder Gasgemisches beispielsweise zum Betrieb einer Membranpumpe verwendet, welche eine pulsierende Förderung der Flüssigkeit bewirkt.

In einer weiteren Ausführungsform der Erfindung wird die innere Energie des Gases oder Gasgemisches separat, vorzugsweise jedoch kombiniert in einem Stoff- und Energiekreislauf als Kraft-Wärme-Kopplung genutzt. Die Kraft-Wärme-Kopplung wird in der Energiewirtschaft, insbesondere bei Kraftwerken als gleichzeitige Gewinnung von mechanischer Energie, welche unmittelbar in Strom umgewandelt wird und nutzbarer Wärme für Heizzwecke verstanden. Im Sinne der vorliegenden Patentanmeldung wird entsprechend die Gewinnung mechanischer Arbeit zum Betrieb der Membranpumpen und die Nutzung der Verdunstungs-/Expansionswärme durch die Entspannung des eingesetzten Gases oder Gasgemisches verstanden.

In einer weiteren Ausführungsform der Erfindung wird das Gas oder Gasgemisch nach Einspeisung in die Flüssigkeit, Suspension oder Emulsion teilweise oder vollständig in der Flüssigkeit, Suspension oder Emulsion umgesetzt und/oder in der Flüssigkeit, Suspension oder Emulsion gelöst. Dies insbesondere vorteilhaft, wenn das eingespeiste Gas, welches eine Pulsation der Flüssigkeit bewirkt, als Substrat für die im Photobioreaktor kultivierten Mikroorganismen dient. So kann beispielsweise ein CO₂-enthaltendes Gas oder Gasgemisch zur Pulsation eingespeist werden, wobei dieses etwa durch Mikroalgen in der Lichtreaktion der Photosynthese zu O₂ verstoffwechselt wird.

In einer weiteren Ausführungsform der Erfindung wird das Gas oder Gasgemisch in einen Photobioreaktor eingespeist. Als Photoreaktor oder auch Photobioreaktor wird dabei ein Bioreaktor zur Kultivierung und Produktion von phototrophen ein- oder mehrzelligen Organismen wie Algen, Cyanobakterien, Moospflanzen oder pflanzliche Zellkulturen bezeichnet.

In einer weiteren Ausführungsform der Erfindung wird das Gas oder Gasgemisch in einen Photobioreaktor mit Mikroalgen eingespeist. Die Bezeichnung "Alge" umfasst im weiteren Sinn im Wasser lebende, eukaryotische, pflanzenartige Lebewesen, die Photosynthese betreiben, jedoch nicht zu den eigentlichen Pflanzen gehören. Im engeren Sinne werden damit zahlreiche Protistengruppen bezeichnet. Zu den Algen gehören sowohl mikroskopisch kleine einzellige, als auch mehrzellige, zum Teil riesige Lebewesen. Als Mikroorganismen werden definitionsgemäß nur ein- bis wenigzellige Algen angesehen, welche als Mikroalgen bezeichnet werden. Diese Mikroalgen betreiben wie alle Algen Photosynthese, wobei sie Licht als Energiequelle nutzen und Kohlenstoff-autotroph sind. Weitere Vertreter Kohlenstoff-(photo)-autotropher Lebewesen sind Schwefelpurpurbakterien oder grüne Schwefelbakterien.

In einer weiteren Ausführungsform der Erfindung wird Kohlenstoffdioxid eingespeist. Dies ist insbesondere bei der Kultivierung von Mikroalgen vorteilhaft, da diese Kohlenstoffdioxid in Biomasse umwandeln. Dadurch ist eine umweltfreundliche Umsetzung von Kohlenstoffdioxid bei gleichzeitiger Erzeugung von Biomasse möglich.

In einer weiteren Ausführungsform der Erfindung wird ein Gas oder Gasgemisch umfassend Kohlenstoffdioxid eingespeist. Bei dem eingespeisten Gasgemisch kann es sich dabei etwa um ein Rauchgas handeln. Die durch die adiabatische Entspannung gebildete Entspannungswärme kann dabei optional zur Kühlung des Kühlwassers bei Verwendung eines Doppelschlauchsystems genutzt werden.

In einer weiteren Ausführungsform der Erfindung wird neben dem Gas oder Gasgemisch, welches zu Pulsation eingespeist wird, ein weiteres Gas oder Gasgemisch umfassend Kohlenstoffdioxid eingespeist. Das weitere Gas oder Gasgemisch, umfassend Kohlenstoffdioxid, dient dabei als Substrat zur Lichtreaktion der phototrophen Mikroorganismen. Das zur Pulsation eingespeiste Gas oder Gasgemisch kann dabei beispielsweise ein inertes Gas oder Gasgemisch, wie etwa Luft oder eine Edelgas sein. Die durch die adiabatische Entspannung gebildete Entspannungswärme kann dabei optional zur Kühlung des Kühlwassers bei Verwendung eines Doppelschlauchsystems genutzt werden.

In einer Ausgestaltung der vorbeschriebenen Ausführungsform weist das zur Pulsation eingespeiste Gas oder Gasgemisch umfassend Kohlenstoffdioxid eine hohe innere Energie auf und wird zur pulsierenden Förderung der Flüssigkeit mittels Membranpumpe eingesetzt.

In einer alternativen Ausgestaltung der vorbeschriebenen Ausführungsform weist das zur Pulsation eingespeiste Gas oder Gasgemisch eine hohe innere Energie auf und wird zur pulsierenden Förderung der Flüssigkeit mittels Membranpumpe eingesetzt. Daneben wird ein weitere Gas oder Gasgemisch in den Photobioreaktor eingespeist, welches Kohlenstoffdioxid umfasst. Die Zugabe des weiteren Gases oder Gasgemisches kann dabei kontinuierlich mit einer gleichbleibenden Rate erfolgen.

In einer weiteren Ausführungsform der Erfindung wird das Gas oder Gasgemisch an einer Unterseite des Reaktors eingespeist und steigt vertikal durch die Flüssigkeit, Suspension oder Emulsion entgegen der Schwerkraftrichtung nach oben. Zur Ausbildung des gewünschten Effekts ist der Reaktor, welcher vorzugsweise als Schlauchreaktor ausgebildet ist, im Gegensatz zum Air-Lift-Prinzip nicht vertikal, sondern schräg ausgebildet, wobei die Einspeisung des Gases nicht in fein verteilter Form, sondern in einem Stoß als Gasblase erfolgt. Unter "schräg" wird im Sinne der vorliegenden Erfindung ein Schlauch mit einem Winkel zur Horizontalen von 0°< α <90°, vorzugsweise 0°< α <10°, besonders bevorzugt 0°< α < 5°verstanden, welcher helikal auf ein kegelstumpfförmig ausgebildetes Reaktorgestell gewickelt ist, verstanden. Durch die Einspeisung des Gases oder Gasgemisches an der Reaktorunterseite und deren Aufsteigen in einem schrägen Reaktorsystem entgegen der Schwerkraftrichtung werden Totzonen im Reaktor vermieden und damit eine gleichmäßig pulsierende Einspeisung ermöglicht, dadurch lässt sich zudem eine genauere Kontrolle der Pulsation und damit einer turbulenten Durchmischung der Flüssigkeit, Suspension oder Emulsion erzielen.

In einer weiteren Ausführungsform der Erfindung wird die strömende Flüssigkeit, Suspension oder Emulsion im Reaktor entgegen der Schwerkraft mit 0,35-0,50 m/s, vorzugsweise mit 0,40 m/s durch den Photobioreaktor gepumpt. Die strömende Flüssigkeit, Suspension oder Emulsion im Reaktor wird dabei vorzugsweise mittels einer Membranpumpe gepumpt.

In einer weiteren Ausführungsform der Erfindung beträgt die eingespeiste Gasmenge des pulsierend eingespeisten Gases oder Gasgemisches unter Normbedingungen (20°C, 101325 Pa) je nach Feststoffkonzentration und/oder Verschmutzungsgrad, vorzugsweise ab einer Algenkonzentration von 6 g/l in der Flüssigkeit, Suspension oder Emulsion, 0,00-1,00 Liter/s, vorzugsweise 0,00-0,10 Liter/s, besonders bevorzugt 0,00-0,01 Liter/s.

Gegenstand der vorliegenden Erfindung ist auch ein Verfahren zum Suspendieren oder Emulgieren von Gemischen in einem Reaktor, wobei mittels einer Entspannung eines eingespeisten Gases oder Gasgemisches eine Triebkraft erzeugt wird. Die dabei gewonnene Triebkraft erzeugt in der Flüssigkeit, Suspension oder Emulsion starke Turbulenzen, wodurch vorteilhafterweise eine schonende, stoff- und energieeffiziente Homogenisierung der Suspensions- oder Emulsionsgemische erreicht wird. Als starke Turbulenzen werden im Sinne der Erfindung nicht laminare, also turbulente Strömungen (RE > 2300) in der Flüssigkeit, Suspension oder Emulsion verstanden, die durch den Gaseintrag gezielt verstärkt werden und damit beispielsweise Wandanhaftungen durch intensive (Phasen)-Durchmischung verhindern können.

Die vorbeschriebenen Verfahrensschritte sind insbesondere vorteilhaft zur Vermeidung von Biobelagbildung in einem Reaktor. Beispielweise wird durch die, aufgrund der Gaseinspeisung in den Reaktor entstehenden Turbulenzen eine Wandanhaftung von Feststoffteilchen, Mikroorganismen und dergleichen im Reaktor unterbunden. Damit werden Reaktorstillstandzeiten minimiert und notwendige Reinigungstätigkeiten auf ein Mindestmaß reduziert. Zugleich können durch die Unterbindung der Wandanhaftung von Mikroorganismen höhere Biotrockenmassekonzentrationen erzielt werden, was letztlich zu einer effektiveren Prozessführung mit deutlich höheren Raum-Zeit-Ausbeuten führt.

Die Aufgabe wird auch durch eine Vorrichtung zur Durchführung des vorbeschriebenen Verfahrens gelöst. Die Vorrichtung umfasst dabei einen Reaktor, wobei an der Unterseite des Reaktors eine Einrichtung zur Einspeisung des Gases oder Gasgemisches vorgesehen ist. Dadurch steigt das eingespeiste Gas oder Gasgemisch durch die Flüssigkeit, Suspension oder Emulsion entgegen der Schwerkraftrichtung nach oben, wobei durch die schräge Anordnung des Reaktors und die gepulste Einspeisung des Gases in nicht fein verteilter Form ein Gasblasenszenario entsteht, durch welches die Flüssigkeit durch schlagartiges Zurückströmen in Schwerkraftrichtung die sich gebildeten Gasräume zerschlägt. Infolgedessen werden bei pulsierender Einspeisung des Gases oder Gasgemisches Turbulenzen in der Flüssigkeit, Suspension oder Emulsion erzeugt, wodurch eine intensive Durchmischung der Flüssigkeit, Suspension oder Emulsion gegeben ist. Infolgedessen wird eine Biobelagbildung an der Wandung des Reaktors unterbunden. Zudem wird durch die pulsierende Einspeisung des Gases oder Gasgemisches ein Absetzen von Feststoffen bzw. eine Phasentrennung unterbunden. Durch die Einspeisung des Gases oder Gasgemisches an der Reaktorunterseite und deren Aufsteigen vertikal entgegen der Schwerkraft- und Strömungsrichtung werden Totzonen im Reaktor vermieden und damit eine gleichmäßige, pulsierende Einspeisung ermöglicht. Dadurch lässt sich zudem eine genauere Kontrolle der Pulsation und mithin der Durchmischung der Flüssigkeit, Suspension oder Emulsion erzielen. Je stärker die Steigung im Reaktor, desto höhere Turbulenzen können durch die Gas-Pulsation erzielt werden, wobei der Winkel α der Reaktorsteigung nicht größer als 90°, vorzugsweise <10°, besonders bevorzugt <5° betragen sollte. Aufgrund der Nutzung von Schwerkraft und Auftrieb wird eine material- und energieeffiziente Verfahrensweise ermöglicht.

Zudem ist die vorbeschriebene Vorrichtung vorteilhaft zur Kultivierung von Mikroalgen, da infolge der pulsierenden Einspeisung des Gases oder Gasgemisches eine fortwährende Durchmischung der Algenkultur gewährleistet und damit eine ausreichende Belichtung der Algen in der Kultur ermöglicht wird. Dadurch können im Vergleich zu bekannten Photobioreaktoren höhere Mikroalgenkonzentrationen im Bereich ≥ 7 g/l eingesetzt werden, was einer Steigerung von über 40% entspricht.

In einer weiteren Ausführungsform der Erfindung ist der Reaktor in seiner Geometrie beliebig ausgebildet, wobei dieser bevorzugt rund (polyedrische Grundfläche), besonders bevorzugt als Rohr- und/oder Schlauch-/Doppelschlauchreaktor ausgebildet ist. Zur Minimierung von Anhaftungen ist auch die Minimierung von (Strömungs)-Totzonen im System sinnvoll, daher sollten kreisförmige Geometrien, insbesondere der Fördersysteme, wie z. B. in Schläuchen, Rohren oder Schlauchreaktoren/Schlauchphotobioreaktoren bevorzugt eingesetzt werden. Diese Geometrie gestattet auch die flexible, schräge Anordnung des Reaktionssystems in einem Winkel von 0°< α <90°, vorzugsweise 0°< α <10°, besonders bevorzugt 0°< α < 5° gegenüber der Horizontalen.

Die erfindungsgemäße Lösung betrifft somit ein Verfahren zur Erzeugung von Pulsationen durch Einsatz von Gasen und/oder Gasgemischen hoher innerer Energie, insbesondere in verdichtetem, zumindest teilkondensiertem oder zumindest teilgefrorenem Zustand. Dabei wird die innere Energie der Gase bzw. Gasgemische zum Antrieb einer Membranpumpe und damit zur Erzeugung eines Impulses genutzt. Die Pulsation bewirkt eine intensive Durchmischung der Flüssigkeit, Suspension oder Emulsion durch gezielt erzeugte Turbulenzen mit den eingespeisten Gasen oder Gasgemischen im Reaktionsraum und verhindert beispielsweise ein Absetzen von Suspensionen oder Wandanhaftung fester Teilchen. Dabei wird die Energie des eingesetzten, verdichteten, kondensierten und/oder gefrorenen Gases stoff- und energiewirtschaftlich optimal genutzt, indem der Flüssigkeitstransport, die Erzeugung von Strömungen/Turbulenzen durch Pulsationen, Kühlkapazität und auch der Reaktionspartner gleichzeitig oder separat durch das eingesetzte Gas/Gasgemisch zur Verfügung gestellt werden. Der genannte Pulsationseffekt kann verstärkt werden, wenn zur Gaseinspeisung die Förderung der Flüssigkeit ebenfalls pulsierend, z. B. mit einer Membranpumpe erfolgt.

Vorteilhafterweise sind bei dieser Verfahrensweise Gase oder Gasgemische mit hoher innerer Energie, d. h. in verdichtetem, (teil-)kondensierten und/oder (teil-)gefrorenen Zustand einzusetzen. Die Gasart richtet sich insbesondere nach der durchzuführenden Reaktion, Verfügbarkeit, Preis, Materialverträglichkeit u. a., prinzipiell können aber beliebige Gase/Gasgemische eingesetzt werden.

Die Gasmenge richtet sich z. B. nach dem Luftverbrauch der Membranpumpen (Förderhöhe, Fördervolumina, gewünschte Dichteunterschiede zwischen den Phasen, Absetzverhalten, etc.), von Gleichgewichtsbedingungen (Konzentration für die Reaktion/Löslichkeit, pH-Wert, Reaktivität oder Reaktionsgeschwindigkeit) sowie der gewünschten Turbulenzen durch gezielte Pulsation des Gases in die (Reaktions)-Lösung.

Hinsichtlich des Reaktors bestehen generell keine Begrenzungen, es können beliebige Geometrien des Reaktors bzw. des Fördersystems gewählt werden. Zur Minimierung von Anhaftungen ist auch die Minimierung von (Strömungs)-Totzonen im System sinnvoll, daher sollten kreisförmige Geometrien insbesondere der Fördersysteme, wie z. B. in Schläuchen oder Schlauchreaktoren/Schlauchphotobioreaktoren bevorzugt eingesetzt werden. Erfindungsgemäß erfolgt die Gaseinspeisung an der Reaktorunterseite, deshalb ist eine gegenüber der Horizontalen oder steigende Strömungsrichtung notwendig.

In einer weiteren Ausführungsform der Erfindung ist der Photobioreaktor als Schlauch- oder Doppelschlauchreaktor ausgeführt, wobei die Schläuche transparent oder transluzent sind und vorzugsweise aus einem flexiblen Material bestehen. Dies ist insbesondere vorteilhaft, da durch die flexible Gestaltung der Schläuche mögliche Belastungen des Materials infolge der Pulsation vermieden werden. Vorzugsweise sind die Schläuche aus einem flexiblen Polymer, wie beispielsweise PET ausgeführt. Die transparente oder transluzente Ausgestaltung der Schläuche ist dabei essentiell, um eine Kultivierung der phototrophen Mikroorganismen zu gewährleisten.

In einer weiteren Ausführungsform der Erfindung weist der Photobioreaktor eine kegelstumpfförmige Kernstruktur und eine oder mehrere transparente oder transluzente Schläuche auf, welche um die Außenseite und/oder Innenseite der Kernstruktur helikal aufgewickelt sind. Dabei umfasst der transparente oder transluzente Schlauch mindestens zwei Kammern, von denen mindestens eine von dem Kultivierungsmedium durchströmt wird und mindestens eine von einem Wärmetauschmedium durchströmt wird. Im Gegensatz Air-Lift-Prinzip wird erfindungsgemäß keine Gasverteilung, sondern eine Blase durch einen Druckstoß, beispielsweise über ein Magnetventil ohne Verteilungsvorrichtung, eingespeist, woraus sich in Kombination mit der Schrägstellung des Reaktors und der Strömungsgeschwindigkeit der Flüssigkeit nachfolgend beschriebenes Szenario ergibt.

In einer weiteren Ausführungsform der Erfindung wird zur Einspeisung des Gases ein direkt gesteuertes Magnetventil verwendet, mit deren Hilfe ein nicht fein verteilter Gasstoß erzeugt wird.

Das beschriebene Verfahren ist vielseitig und universell in vielen technischen Bereichen einsetzbar, z.B. überall dort wo Flüssigkeiten/Reaktionsmedien/Suspensionen gefördert werden müssen, in denen Gase mit diesen reagieren oder gelöst werden müssen, zur Abführung von (außen eingestrahlter) Wärme bei exothermen Reaktionen/Lösung von Gasen oder Gasgemischen.

### Beschreibung des Pulsationseffektes

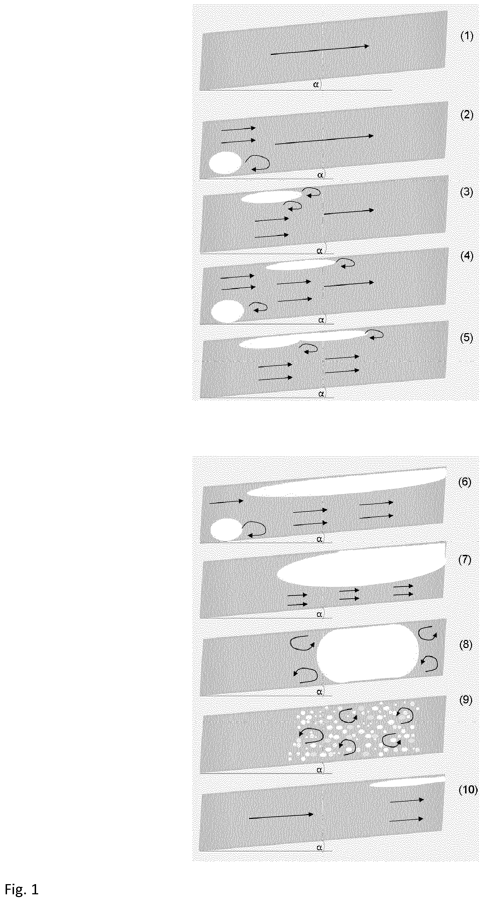

Nachfolgend soll anhand der **Fig. 1** die erfindungsgemäße Erzeugung von Turbulenzen in der Reaktionslösung oder Suspension beschrieben werden. Dabei wird die Flüssigkeit, Suspension oder Emulsion mittels Membranpumpe pulsierend durch ein Rohr- oder Schlauchsystem gefördert, welches schräg mit einem von der Horizontalen abweichenden Winkel α angeordnet ist (1). Unterhalb des Reaktor-, Rohr- oder Schlauchsystems erfolgt zusätzlich die Einspeisung eines Gases oder Gasgemisches. Der Gasdruck und die Gasmenge richten sich nach Berstdruck des Reaktors, Verschmutzungsgrad und/oder Feststoffgehalt der Flüssigkeit, Suspension oder Emulsion (2). Die eingespeisten Gasblasen bewegen sich langsam zur Strömungsrichtung und im oberen Teil des Rohr- oder Schlauchsystems. Aufgrund der Querschnittsverengung nimmt die Strömungsgeschwindigkeit der Flüssigkeit unterhalb der Gasphase zu (3). Durch stetes Nachströmen von Gas entsteht eine Gasblase im Rohr- oder Schlauchsystem, die Strömungsgeschwindigkeit der Flüssigkeit ist über einen größeren Abschnitt hoch (4-7). Wenn sich genügend Gas gesammelt hat, entsteht ein Gaspfropf, die Flüssigkeit wird dabei kurzzeitig nicht weiter gefördert (8). Wenn der Gegendruck der Flüssigkeit zu hoch wird, erfolgt schlagartig das Zurückströmen der Flüssigkeit in den gasgefüllten Raum. Die hohe Strömungsgeschwindigkeit, sowie zahlreiche kleine Gasbläschen bewirken sehr starke Turbulenzen. An der Wandung anhaftende Feststoffe werden gelöst, sowie sich absetzende Partikel wieder aufgewirbelt (9). Nachdem sich aus den kleinen Gasbläschen wieder eine größere Gasblase gebildet hat (10), beginnt der Vorgang an einer anderen Stelle entlang der Strömungsrichtung erneut (siehe Schritt 2).

Erfindungsgemäß kann dieses Verfahren einer gezielten, pulsierenden Gaszugabe zu einer pulsierend geförderten Flüssigkeit sowohl zur Verhinderung des Absetzens von Suspensionen bzw. Verhinderung von Anhaftungen an der Reaktor-/Rohr-/Schlauchwand, als auch zum Reinigen dieser effizient genutzt werden. Die genannte Technologie ist damit sowohl zur prophylaktischen/präventiven, als auch zur nachträglichen Reinigung von Anlagen oder Anlagenteilen geeignet.

In einer weiteren Ausführungsform der Erfindung wird als Gas Kohlendioxid verwendet.

In einer weiteren Ausführungsform der Erfindung wird verdichtetes, kondensiertes und/oder gefrorenes Kohlendioxid in Reaktoren, vorzugsweise Bioreaktoren verwendet.

Die vorbeschriebenen Verfahren und Vorrichtungen werden in Flüssigkeit, Suspensionen oder Emulsionen gleichwertig angewendet. Sofern in der Beschreibung nur einer der Begriffe verwendet wird, stellt dies keine explizite Einschränkung dar, sondern lediglich eine exemplarische Aufzählung die jedoch die anderen Begriffe mit umfassen soll.

Wesentlich für die Ausbildung des Pulsationseffektes ist die genaue Einstellung der Parameter eingespeiste Gasmenge, Strömungsgeschwindigkeit der Flüssigkeit, Suspension oder Emulsion sowie der Steigungswinkel der Schläuche des Reaktors, wobei in Abhängigkeit der einzelnen Parameter ein Optimum zwischen Strömungsgeschwindigkeit der Flüssigkeit, eingespeiste Gasmenge und Anstellwinkel des Reaktors einzustellen ist.

Zur Lösung der Aufgabe ist es auch vorgesehen, die vorbeschriebenen Ausführungsformen zweckmäßig miteinander zu kombinieren.

Nachfolgend soll die Erfindung anhand einiger Ausführungsbeispiele und der zugehörigen Figuren eingehender erläutert werden. Die Ausführungsbeispiele sollen die Erfindung beschreiben ohne sich auf diese zu beschränken.

Es zeigen die
**Fig.1** eine schematische Darstellung des Pulsationseffektes in einem Schlauchreaktor, in
**Fig.2** ein Verfahrensfließschema beim Einsatz verdichteter/ kondensierter Gase, in
**Fig.3** ein Verfahrensfließschema beim Einsatz gefrorener Gase, z. B. Trockeneis, in
**Fig.4** ein qualitatives Energieflußschema und in
**Fig.5** ein quantitatives Energieschema beim Einsatz von flüssigem Kohlenstoffdioxid.

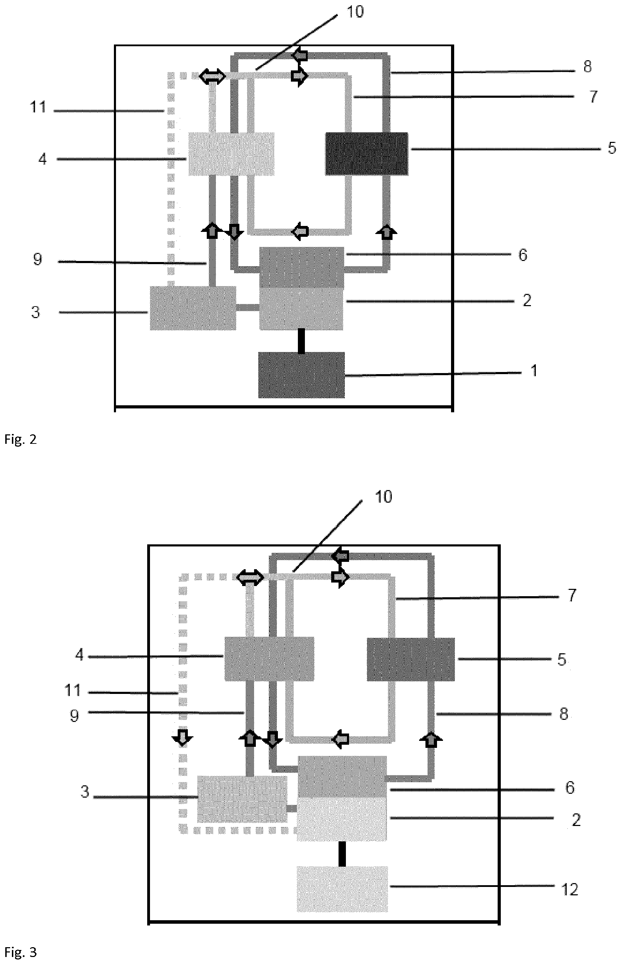

In einem ersten Ausführungsbeispiel besteht entsprechend des in der **Fig. 2** dargestellten Verfahrensfließschematas die Anlage aus einem Vorratstank 1, einer Verdampfer-Einheit 2 mit Wärmetauscher 6, einem Lagertank 3, Membranpumpen 4 für Reaktionslösung 7 und Wärmeüberträgermedium 8, sowie dem Reaktor 5. Hinter der Membranpumpe 4 sind eine Gas-Dosierung 9, sowie eine Rückführungsleitung 11 vorgesehen. Die Gaseinspeisung in die Reaktionslösung 8 erfolgt über eine nicht näher dargestellte Einspeiseeinrichtung 10.

In einem weiteren Ausführungsbeispiel ist in der **Fig. 3** ein der Fig. 2 analoges Verfahrensfließschemata dargestellt, wobei der Vorratstank 12 Trockeneis enthält, welches in einem CO₂-Verdampfer 2 erwärmt wird, wobei der CO₂-Verdampfer 2 mit einem Wärmetauscher 6 verbunden ist und eine Kühlung des Wärmeübertragungsmediums 8 und damit der Reaktionslösung 6 im Reaktor 5 erfolgt. Das CO₂ wird weiterhin in einem Lagertank 3 gespeichert mittels der Membranpumpen 4 über eine nicht näher dargestellte Einspeiseeinrichtung 10 in die Reaktionslösung 7 eingebracht. Erfindungsgemäß erfolgt die Einspeisung in die Reaktionslösung 7 pulsierend, sodass in der Reaktionslösung 7 Turbulenzen erzeugt werden, welche neben eine Durchmischung der Reaktionslösung auch eine Wandanhaftung von Feststoffen an der Reaktorwandung unterbindet. Weiterhin ist eine Rückführungsleitung 11 vorgesehen, welche von der Membranpumpe 4 in den CO₂-Verdampfer 2 führt.

Der Vorgang findet bei einer gleichmäßigen Geometrie an jeder Stelle des Rohr- oder Schlauchsystems statt, allerdings ist Rohr-/Schlauchquerschnitt, Strömungsgeschwindigkeit, Pulsation und die eingespeiste Gasmenge individuell auf das System abzustimmen. Bei einem Rohrquerschnitt von 42 mm, einer Strömungsgeschwindigkeit der Flüssigkeit von 0,45 m/s (2 m³/h), einer Pulsfrequenz der Membranpumpe von 2 Hz, sowie einer eingespeisten Gasmenge von etwa 100 ml / 10 s kann der genannte Effekt beobachtet werden.

In einem weiteren Ausführungsbeispiel wurde ein Vergleichsversuch mit einer Mikroalgenkonzentration über 7 g/l in einem herkömmlichen Schlauch-Photobioreaktor durchgeführt, wobei selbst eine Verdopplung der Strömungsgeschwindigkeit im Rohr-/Schlauchsystem ein Absetzen nicht verhindern konnte. Dagegen waren bei der Anwendung der Gas-Flüssigkeits-Pulsation bei einer Halbierung der Strömungsgeschwindigkeiten keine Absetz- oder Anhaftungseffekte zu beobachten.

Aus den genannten Effekten der Verhinderung von Anhaftungen/Absetzen von Suspensionen bei deutlich geringerer Strömungsgeschwindigkeit, ergibt sich eine äußerst energie- und stoffeffiziente Verfahrensweise.

In einem weiteren Ausführungsbeispiel wurde eine weitere Optimierung im Aufbau eines Gas-Stoffkreislaufs (vgl. Fig. 2 und 3) erzielt. Entscheidend ist die Nutzung der hohen inneren Energie des eingesetzten, verdichteten, (teil)-verflüssigten oder (teil)-gefrorenen Gases oder Gasgemisches. Durch adiabatische Entspannung des Gases erfolgt dessen Abkühlung, womit z. B. Kühlwasser gekühlt werden kann. Das entspannte Gas (p > 1,5 bar) wird nun zum Betrieb der Membranpumpen, welche sowohl die Flüssigkeit/Suspension/Emulsion, als auch das Kühlwasser durch den Reaktor fördern, verwendet. Das aus den Membranpumpen ausgestoßene Gas (p > 1,0 bar) wird nun in definierter Menge und Zeit pulsierend zur Flüssigkeit gegeben. Damit wird nicht nur ein effizienter Stoffkreislauf (Gas befördert Flüssigkeiten/Suspensionen/Emulsionen/Kühlwasser, erzeugt durch pulsierenden Einsatz Turbulenzen, nimmt an der Reaktion teil, etc.), sondern auch eine optimale Energieausnutzung (Kühlung des Kühlwassers durch Entspannen des Gases) mit den zur Verfügung stehenden Ressourcen erhalten.

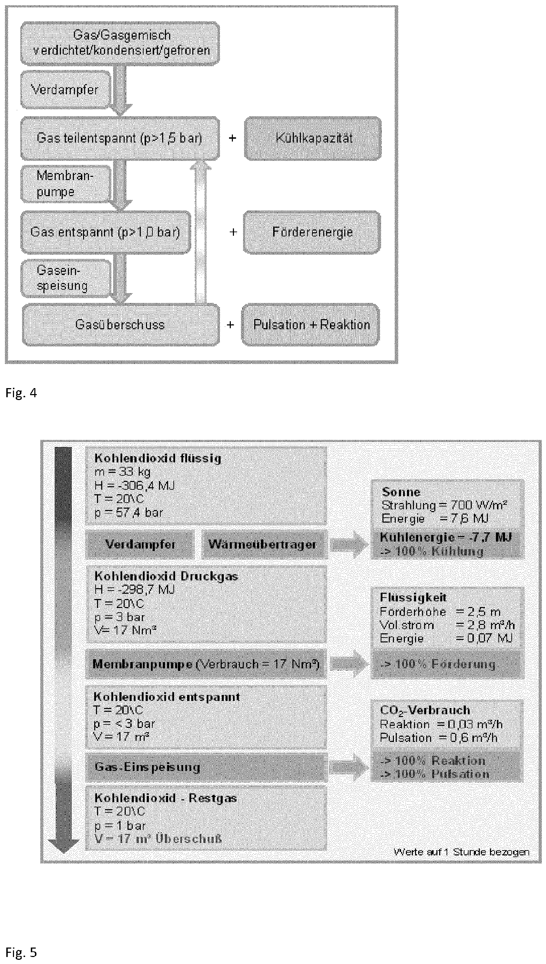

In einem weiteren Ausführungsbeispiel ist in der **Fig. 4** schematisch dargestellt, wie das Gas/Gasgemisch seine innere Energie im Verfahrenskreislauf ändert. Dabei weist das Gas bzw. Gasgemisch zunähst eine hohe innere Energie auf, da es verdichte, kondensiert oder gefroren vorliegt. Das Gas bzw. Gasgemisch wird dann über einen Verdampfer 2 in den Verfahrenskreislauf eingebracht, wobei es teilentspannt wird. Bei dieser Entspannung wird durch die Abkühlung des Gases oder Gasgemisches beim Übertritt von einem Bereich hohen Druckes in einen Bereich niederen Druckes Kälte frei, welche erfindungsgemäß zur Kühlung verwendet wird. Das Gas bzw. Gasgemisch wird dann mittels der Membranpumpe 4 und der Einspeiseeinrichtung 10 in die Reaktionslösung 7 eingebracht, wo aufgrund des erfindungsgemäßen pulsierenden Eintrags Turbulenzen erzeugt werden (Re > 2300), was zu einer Durchmischung der Reaktionslösung 7 und zur Unterbindung von Wandanhaftungen führt. Zudem kann das eingespeiste Gas bzw. Gasgemisch als Edukt in die Reaktionslösung 7 eingebracht werden, wo es dann in einer Reaktion zu einem Produkt, wie etwa Biomasse umgesetzt werden kann. Daneben wird vorteilhafterweise die innere Energie des Gases oder Gasgemisches separat, vorzugsweise jedoch kombiniert in einem Stoff- und Energiekreislauf als Kraft-Wärme-Kopplung genutzt. Die Kraft-Wärme-Kopplung bezeichnet hierbei die Gewinnung mechanischer Arbeit zum Betrieb der Membranpumpen 4 und die Nutzung der Verdunstungs-/Expansionswärme durch die Entspannung des eingesetzten Gases oder Gasgemisches verstanden.

In einem weiteren Ausführungsbeispiel sind in **Fig. 5** exemplarisch mit dem erfindungsgemäßen Verfahren erreichte Werte für die Einspeisung von Kohlenstoffdioxid dargestellt, wobei die dargestellten Werte sich auf Werte pro Stunde beziehen.

In einem weiteren Ausführungsbeispiel wurde der Pulsationseffekt an einer Vorrichtung getestet, die zur Kultivierung von Mikroalgen dient. Die Vorrichtung umfasst einen Photobioreaktor, welcher kegelstumpfförmig ausgeführt ist und eine Höhe ca. 2,0 m aufweist. Der Durchmesser dieses kegelstumpfförmigen Photobioreaktors beträgt unten ca. 2,5m und oben ca. 1,5m, wobei dieser aus einem transluzenten, helikal aufgewickelten Doppelschlauchsystem mit einem Schlauchdurchmesser von ca. 50mm, einem Schlauchabstand von ca. 35mm, einer Länge von ca. 150m bei einem photoaktiven Volumen von ca. 200 L aus Silikon ausgeführt ist. Weiterhin umfasst die Vorrichtung eine Sensorstation zur Bestimmung des pH-Werts, der optischen Dichte, der Temperatur-, des CO₂-Gehalts, einer Durchfluss-Messung, einen Vorlagebehälter mit einem Volumen von ca. 200 I, einem Pumpenstand mit einer Kreisel-/Membranpumpe, sowie verbindenden Rohrleitungen aus PE. Die in den Reaktor eingespeisten Gase können am Vorlagebehälter, an der Unterseite des Reaktors oder im verbindenden Rohrleitungssystem über Ventile zudosiert werden. Die Strömungsgeschwindigkeiten der Suspension liegen im Bereich 0,35-0,50 m/s.

In einem weiteren Ausführungsbeispiel wurde die vorbeschriebene Vorrichtung ohne Pulsation im Normalbetrieb verwendet. Dabei zeigte sich, dass an den Lichteintrittsflächen der PE-Schläuche ab einer Biotrockenmassekonzentration von 3-4 g/L erste Ablagerungen beobachtet werden konnten. Beim Einsatz von Silikonschläuchen verhindert das Silikon als Schlauchmaterial bedingt einen Bewuchs, sodass erst ab 6-7 g/L Biotrockenmasse erste Ablagerungen beobachtet werden können. Entsprechend der angegebenen Konzentrationen erfolgt durch fehlendes Licht für die Photosynthese kein weiteres Mikroalgenwachstum. Nach erfolgter Ernte wird der Reaktor, z.B. mit Wasserstoff-peroxid gereinigt, wobei die Reinigungszeit unabhängig vom eingesetzten Schlauchmaterial 4-6 Tage betrug.

In einem weiteren Ausführungsbeispiel wurde die vorbeschriebene Vorrichtung mit Pulsation verwendet. Dazu wurde die Mikroalge *Scenedesmus rubescens* 20 Tage kultiviert. Die Biotrockenmasse und der Nährstoffgehalt der Suspension wurden täglich gemessen. Ab einer Biotrockenmassekonzentration von etwa 5 g/L (nach ungefähr 1 Woche) wurde erneut Nährmedium zugegeben und die Pulsation (Pulszeit 1 s, Pulsfrequenz 3 s, Pulsationsdauer 10 min/h) bis zum Erreichen einer Maximalkonzentration von ca. 9 g/L nach insgesamt 14 Tagen eingeschaltet. Danach wurden die Mikroalgen per Separator abgetrennt.

Dabei zeigte sich, dass die Mikroalgenproduktivität in der 1. Woche mit ca. 0,5 g/L/d der erwarteten Zuwachsrate entspricht. Während bei Nichtanwendung des Pulsationsprinzips die Produktivität bei Mikroalgen-konzentrationen von etwa 5-6 g/L, also nach etwa 1 Woche stagniert bzw. Biomasse in der Folgezeit sogar verbraucht werden wurde, wurde bei Anwendung der Pulsation bis Konzentrationen von ca. 9 g/L Biotrockenmasse kein Produktivitätsabfall registriert.

Dabei zeigte sich weiterhin, dass deutlich höhere Endkonzentrationen in der Biotrockenmasse (+30%) aufgrund Minimierung der Biobelagbildung durch den Einsatz der Pulstechnik erzielt werden konnten. Hinsichtlich der sich anschließenden Fest-Flüssig-Trennung zur Gewinnung der Mikroalgen kann diese aufgrund höherer Feststoffkonzentrationen deutlich effizienter durchgeführt werden. Da die Abtrennung der Mikroalgen aus der Suspension einen erheblichen Kostenfaktor darstellt, bietet die Pulsationstechnologie die Möglichkeit einer wirtschaftlichen Mikroalgenproduktion in industriellem Maßstab.

In einem weiteren Ausführungsbeispiel wurde die vorbeschriebene Kultivierung ohne Pulsation durchgeführt. Danach erfolgte die Reinigung der Vorrichtung. Dabei wurde zunächst die komplette Mikroalgensuspension entfernt und die Vorrichtung mit ca. 3%iger Wasserstoffperoxidlösung befüllt. Die noch in der Vorrichtung und an den Schlauchwandungen befindliche Biomasse wurde dadurch abgetötet. Erst durch Einsatz der Pulsationstechnik (Pulszeit 1 s, Pulsfrequenz 3 s, Pulsationsdauer 30 min/h) wurde auch hartnäckig festsitzender Biobelag von den Wandungen gelöst. Im Ergebnis war die Vorrichtung nach etwa 3 Tagen wieder betriebsbereit.

In einem weiteren Ausführungsbeispiel wurde die vorbeschriebene Kultivierung mit Pulsation durchgeführt. Die danach durchgeführte Reinigung der Vorrichtung ergab, dass die Reinigungszeit des Schlauch-Photobioreaktorsystems durch den Einsatz von Pulsationen um bis zu 50% reduziert und damit die Anlagenverfügbarkeit entsprechend erhöht werden konnte.

## Patentansprüche

1. Verfahren zur gezielten Einspeisung von Gasen oder Gasgemischen in eine strömende Flüssigkeit, Suspension oder Emulsion in einem Photobioreaktor, **dadurch gekennzeichnet, dass** das Gas- oder Gasgemisch in gezielter Menge und/oder zu definierten Zeitpunkten in einem Stoß als Gasblase in eine strömende Flüssigkeit in einem schräg angeordneten Reaktorsystem eingespeist wird, wodurch ein Pulsationseffekt erhalten wird, wobei mittels einer adiabatische Entspannung des eingespeisten Gases oder Gasgemisches eine Triebkraft erzeugt wird, durch die Wandanhaftungen am Reaktor unterbunden werden, wobei Gase oder Gasgemische mit einer hohen inneren Energie eingesetzt werden und wobei durch vollständige oder teilweise Nutzung der inneren Energie des eingespeisten Gases oder Gasgemisches die geförderte Flüssigkeit, Suspension oder Emulsion gekühlt wird.

2. Verfahren nach Anspruch 1, **dadurch gekennzeichnet, dass** die Flüssigkeit, Suspension oder Emulsion pulsierend gefördert wird.

3. Verfahren nach Anspruch 1 oder 2, **dadurch gekennzeichnet, dass** das Gas oder Gasgemisch verdichtet, zumindest teilweise kondensiert oder zumindest teilweise gefroren eingesetzt wird.

4. Verfahren nach einem der Ansprüche 1 bis 3, **dadurch gekennzeichnet, dass** durch vollständige oder teilweise Nutzung der inneren Energie des eingespeisten Gases oder Gasgemisches, die das Gas oder Gasgemisch aufgrund seiner Verdichtung beinhaltet, die Flüssigkeit pulsierend gefördert wird.

5. Verfahren nach einem der vorhergehenden Ansprüche, **dadurch gekennzeichnet, dass** die innere Energie des Gases oder Gasgemisches separat, vorzugsweise jedoch kombiniert in einem Stoff- und Energiekreislauf als Kraft-Wärme-Kopplung genutzt wird.

6. Verfahren nach einem der vorhergehenden Ansprüche, **dadurch gekennzeichnet, dass** das Gas oder Gasgemisch nach Einspeisung in die Flüssigkeit, Suspension oder Emulsion teilweise oder vollständig in der Flüssigkeit, Suspension oder Emulsion umgesetzt wird und/oder in der Flüssigkeit, Suspension oder Emulsion gelöst wird.

7. Verfahren nach einem der vorhergehenden Ansprüche, **dadurch gekennzeichnet, dass** das Gas oder Gasgemisch in einen Photobioreaktor mit Mikroalgen eingespeist wird.

8. Verfahren nach einem der vorhergehenden Ansprüche, **dadurch gekennzeichnet, dass** das Gas oder Gasgemisch Kohlenstoffdioxid umfasst.

9. Verfahren nach einem der vorhergehenden Ansprüche, **dadurch gekennzeichnet, dass** das Gas oder Gasgemisch an der Unterseite des Photobioreaktors eingespeist wird und vertikal durch die Flüssigkeit, Suspension oder Emulsion entgegen der Schwerkraft- und Strömungsrichtung der Flüssigkeit nach oben steigt.

10. Verfahren nach einem der vorhergehenden Ansprüche, **dadurch gekennzeichnet, dass** die strömende Flüssigkeit Suspension oder Emulsion entgegen der Schwerkraft mit 0,35-0,50 m/s, vorzugsweise mit 0,40 m/s gepumpt wird.

11. Verwendung eines Verfahrens nach einem der Ansprüche 1 bis 10 zur Vermeidung von Biobelagbildung in einem Photobioreaktor.

12. Vorrichtung zur Durchführung eines Verfahrens nach den Ansprüchen 1 bis 10, umfassend einen Photobioreaktor, wobei an der Unterseite des Photobioreaktors eine Einrichtung zur Einspeisung des Gases oder Gasgemisches vorgesehen ist, wodurch das eingespeiste Gas oder Gasgemisch vertikal durch die Flüssigkeit, Suspension oder Emulsion entgegen der Schwerkraft- und Strömungsrichtung der Flüssigkeit nach oben steigt sowie eine Membranpumpe zur pulsierenden Förderung der Flüssigkeit, Suspension oder Emulsion, welche durch die innere Energie des Gases oder Gasgemisches, die das Gas oder Gasgemisch aufgrund seiner Verdichtung beinhaltet, angetrieben wird.

13. Vorrichtung nach Anspruch 12, **dadurch gekennzeichnet, dass** der Photobioreaktor in seiner Geometrie beliebig, bevorzugt rund, besonders bevorzugt als Rohr- und/oder Schlauch-/Doppelschlauchreaktor ausgebildet ist.

14. Vorrichtung nach einem der Ansprüche 12 oder 13, **dadurch gekennzeichnet, dass** der Photobioreaktor als kegelstumpfförmiger Schlauch-/Doppelschlauchreaktor ausgebildet ist, wobei der Schlauch in einem Winkel zur Horizontalen von 0°<α<90°, vorzugsweise 0°<α<10°, besonders bevorzugt 0°<α<5° helikal angeordnet ist.

## Claims

1. A method for feeding gases or gas mixtures into a flowing liquid, suspension or emulsion in a photobioreactor in a specific manner, **characterized in that** the gas or gas mixture is fed with a specific amount and/or at defined points in time into a flowing liquid in a tilted reactor system in one burst in the form of a gas bubble causing a pulsation effect, wherein a propulsive force is created by means of an adiabatic expansion of the fed-in gas or gas mixture preventing material from clinging to the walls of the reactor, wherein the gases or gas mixtures with a high internal energy are used and wherein the liquid, suspension or emulsion that is conveyed is cooled because of the complete or partial use of the internal energy of the fed-in gas or gas mixture.

2. The method according to claim 1, **characterized in that** the liquid, suspension or emulsion is conveyed in a pulsating fashion.

3. The method according to one of the claims 1 or 2, **characterized in that** gas or gas mixture that is used is compressed, at least partially condensed or at least partially frozen.

4. The method according to one of the preceding claims, **characterized in that** the liquid is conveyed in a pulsating fashion because of the complete or partial use of the internal energy of the fed-in gas or gas mixture.

5. The method according to one of the preceding claims, **characterized in that** the internal energy of the gas or gas mixture, that the gas or gas mixture that is employed contains due to its compression, is used separately, but preferably in a combined manner in a material and energy circulation system in the form of combined heat and power generation.

6. The method according to one of the preceding claims, **characterized in that** the gas or gas mixture, after being fed into the liquid, suspension or emulsion, is partially or completely transformed in the liquid, suspension or emulsion and/or dissolved in the liquid, suspension or emulsion.

7. The method according to one of the preceding claims, **characterized in that** the gas or gas mixture is fed into a photobioreactor with micro-algae.

8. The method according to one of the preceding claims, **characterized in that** the gas or gas mixture includes carbon dioxide.

9. The method according to one of the preceding claims, **characterized in that** the gas or gas mixture is fed in from the bottom of the photobioreactor and ascends vertically through the liquid, suspension or emulsion against the direction of gravity and the direction of flow of the liquid.

10. The method according to one of the preceding claims, **characterized in that** the flowing liquid, suspension or emulsion is pumped against the force of gravity at 0.35- 0.50 m/s, preferably at 0.40 m/s.

11. Use of a method according to one of the claims 1 to 10 to avoid bio-film buildup in a photobioreactor.

12. Device for carrying out a method according to claims 1 to 10, comprising a photobioreactor, wherein apparatus for feeding in the gas or gas mixture is provided at the bottom of the photobioreactor, causing the fed-in gas or gas mixture to ascend vertically through the liquid, suspension or emulsion against the direction of gravity and the direction of flow of the liquid, and a diaphragm pump to convey the liquid, suspension or emulsion in a pulsating fashion that is driven by the internal energy of the gas or gas mixture, that the gas or gas mixture that is employed contains due to its compression.

13. The device according to claim 12, **characterized in that** the photobioreactor has an arbitrary geometry, preferably round and especially preferably in the form of a pipe and/or tube / double-tube reactor.

14. The device according to one of the claims 12 or 13, **characterized in that** the photobioreactor is designed as a tube / double-tube reactor with a truncated-cone shape, wherein the tube is helically arranged at an angle vis-a-vis the horizontal plane of 0° < α < 90°, preferably 0° < α < 10°, especially preferably 0° < α < 5°.

## Revendications

1. Procédé destiné à l'introduction ciblée de gaz ou de mélanges gazeux dans un liquide, une suspension ou une émulsion circulant dans un photobioréacteur, **caractérisé en ce que** le gaz ou le mélange gazeux est introduit en une quantité ciblée et/ou à des instants définis d'un seul coup sous la forme d'une bulle de gaz dans un liquide circulant dans un système de réacteur incliné, ce qui permet d'obtenir un effet de pulsation, une détente adiabatique du gaz ou du mélange gazeux introduit générant une force de projectile grâce à laquelle les dépôts adhérant aux parois du réacteur sont détachés, des gaz ou des mélanges gazeux comportant une énergie interne élevée étant utilisés, et le liquide, la suspension ou l'émulsion transporté(e) étant refroidi(e) en utilisant partiellement ou totalement l'énergie interne du gaz ou du mélange gazeux introduit.

2. Procédé selon la revendication 1, **caractérisé en ce que** le liquide, la suspension ou l'émulsion est transporté(e) de manière puisée.

3. Procédé selon la revendication 1 ou 2, **caractérisé en ce que** le gaz ou le mélange gazeux est utilisé sous forme comprimée, au moins partiellement condensée ou au moins partiellement congelée.

4. Procédé selon l'une des revendications 1 à 3, **caractérisé en ce que** le liquide est transporté de manière pulsée en utilisant partiellement ou totalement l'énergie interne du gaz ou du mélange gazeux introduit, le gaz ou le mélange gazeux possédant cette énergie du fait de sa compression.

5. Procédé selon l'une des revendications précédentes, **caractérisé en ce que** l'énergie interne du gaz ou du mélange gazeux est utilisée séparément, mais de préférence de façon combinée dans un circuit de matière et d'énergie en tant que cogénération.

6. Procédé selon l'une des revendications précédentes, **caractérisé en ce que** le gaz ou le mélange gazeux, après avoir été introduit dans le liquide, la suspension ou l'émulsion, est mis à réagir partiellement ou totalement dans le liquide, la suspension ou l'émulsion et/ou est dissous dans le liquide, la suspension ou l'émulsion.

7. Procédé selon l'une des revendications précédentes, **caractérisé en ce que** le gaz ou le mélange gazeux est introduit dans un photobioréacteur à microalgues.

8. Procédé selon l'une des revendications précédentes, **caractérisé en ce que** le gaz ou le mélange gazeux comprend du dioxyde de carbone.

9. Procédé selon l'une des revendications précédentes, **caractérisé en ce que** le gaz ou le mélange gazeux est introduit au niveau de la face inférieure du photobioréacteur et remonte verticalement à travers le liquide, la suspension ou l'émulsion à l'encontre du sens de la gravité et de l'écoulement du liquide.

10. Procédé selon l'une des revendications précédentes, **caractérisé en ce que** le liquide, la suspension ou l'émulsion circulant est pompé(e) à l'encontre de la gravité à 0,35-0,50 m/s, de préférence à 0,40 m/s.

11. Utilisation d'un procédé selon l'une des revendications 1 à 10 pour éviter la formation d'un biofilm dans un photobioréacteur.

12. Dispositif destiné à la mise en œuvre d'un procédé selon les revendications 1 à 10, comprenant un photobioréacteur, un appareil destiné à l'introduction du gaz ou du mélange gazeux étant prévu au niveau de la face inférieure du photobioréacteur, moyennant quoi le gaz ou le mélange gazeux introduit remonte verticalement à travers le liquide, la suspension ou l'émulsion à l'encontre du sens de la gravité et de l'écoulement du liquide, ainsi qu'une pompe à membrane destinée au transport pulsé du liquide, de la suspension ou de l'émulsion, laquelle pompe à membrane est entraînée par l'énergie interne du gaz ou du mélange gazeux, le gaz ou le mélange gazeux possédant cette énergie du fait de sa compression.

13. Dispositif selon la revendication 12, **caractérisé en ce que** le photobioréacteur présente une géométrie arbitraire, de préférence ronde, de manière particulièrement préférée est conçu sous forme de réacteur tubulaire et/ou de réacteur à tube/à tube double.

14. Dispositif selon l'une des revendications 12 ou 13, **caractérisé en ce que** le photobioréacteur est conçu sous la forme d'un réacteur à tube/à tube double tronconique, le tube étant disposé en hélice à un angle par rapport à l'horizontale tel que 0° < α < 90°, de préférence tel que 0° < α < 10°, de manière particulièrement préférée tel que 0° < α < 5°.
